# EUROPÄISCHE PATENTSCHRIFT

(11) **EP 0 905 124 B1**
(45) Veröffentlichungstag und Bekanntmachung des Hinweises auf die Patenterteilung: **12.12.2001**
(21) Anmeldenummer: 98117340.4
(22) Anmeldetag: 12.09.1998
(51) Int. Cl.: C07C 217/90, C07D 213/69

(54) **o-Amino(thio)phenol-carbonsäuren und deren Herstellung**
o-amino(thio)phenol-carboxylic acids and their preparation
Acides o-amino(thio)phenol-carboxyliques et leur préparation

(30) Priorität: 24.09.1997 DE 19742193
(43) Veröffentlichungstag der Anmeldung: 31.03.1999
(73) Patentinhaber: Infineon Technologies AG, 81669 München (DE)
(72) Erfinder: Sezi, Recai, Dr., 91341 Röttenbach (DE); Keitmann, Michael, 91074 Herzogenaurach (DE)
(74) Vertreter: Müller . Hoffmann & Partner Patentanwälte

(56) Entgegenhaltungen:
- EP-A- 0 023 662
- EP-A- 0 264 678

## Beschreibung

Die Erfindung betrifft neue o-Aminophenol-carbonsäuren und o-Aminothiophenol-carbonsäuren, die zusammen kurz auch als o-Amino(thio)phenol-carbonsäuren bezeichnet werden, sowie ein Verfahren zu deren Herstellung.

o-Aminophenol-carbonsauren werden insbesondere zur Herstellung von hochtemperaturstabilen Polymeren, wie Polybenzoxazolen (PBO) bzw. deren Vorstufen, benötigt. Im Vergleich zur Herstellung der Polybenzoxazole bzw. der PBO-Vorstufen aus Bis-o-aminophenolen und Dicarbonsäuren hat die Verwendung von o-Aminophenol-carbonsäuren deutliche Vorteile. So kann eine o-Aminophenol-carbonsaure mit sich selbst umgesetzt werden, d.h. für die Polymerisation ist ein zweites Monomer nicht unbedingt erforderlich. Hierdurch können die Reinheitskontrolle und die Lagerhaltung vereinfacht werden. Außerdem ist die Stöchiometrie bereits vorgegeben, d.h. Fehler bei der Berechnung oder der Einwaage der Reaktanten, wie sie bei der Reaktion von Bis-o-aminophenolen mit Dicarbonsäuren auftreten können, sind bei der Verwendung von o-Aminophenol-carbonsäuren ausgeschlossen. Ferner beeinflußt die Art des verwendeten Monomers in starkem Maße das Eigenschaftsspektrum der damit hergestellten PBO-Vorstufe bzw. des Polybenzoxazols. So werden nicht nur das thermische, elektrische und mechanische Verhalten, sondern auch die Löslichkeit und die Hydrolysestabilität sowie zahlreiche weitere Eigenschaften des Polymers durch das bei der Herstellung verwendete Monomer stark beeinflußt.

PBO-Vorstufen können in Form einer photosensitiven Zusammensetzung kostengünstig auf direktem Weg, d.h. ohne einen Hilfsresist, strukturiert werden. Im Vergleich mit anderen direkt photostrukturierbaren Dielektrika, wie Polyimid (PI) und Benzocyclobuten (BCB), bieten PBO-Vorstufen den Vorteil der positiven Strukturierbarkeit und der wäßrig-alkalischen Entwicklung (siehe EP 0 023 662 B1 und EP 0 264 678 B1). Hierzu müssen die verwendeten PBO-Vorstufen bei der Belichtungswellenlänge weitgehend transparent und im - vorzugsweise metallionenfreien - Entwickler ausreichend löslich sein. Wie die Polyimide haben auch Polybenzoxazole den großen Vorteil, daß sie - im Vergleich zum cyclisierten Endprodukt - als gut lösliche Vorstufe auf ein Substrat aufgebracht und anschließend cyclisiert werden können, wobei die Löslichkeit und damit die Sensibilität gegenüber Lösemitteln und anderen Prozeßchemikalien stark abnimmt.

Für den Einsatz von Polybenzoxazolen in der Mikroelektronik ist neben einer guten Löslichkeit der Vorstufen auch eine niedrige Feuchteaufnahme und ein gutes Planarisierungsvermögen von Vorteil. So können bei der Verwendung eines gut planarisierenden Dielektrikums bei der Herstellung von Bauteilen kostenintensive Schleifprozeduren (Chemical Mechanical Polishing; CMP) vermieden werden.

o-Aminophenol-carbonsäuren sind beispielsweise aus GB 811 758 und GB 1 283 476 bekannt. Bei PBO-Filmen, die aus den bekannten Monomeren hergestellt werden, beträgt die Wasseraufnahme in kochendem Wasser nach 24 h 0,77 %. Auf das Planarisierungsverhalten der hergestellten Polymere nach der Cyclisierung auf dem Substrat oder auf deren Eignung als Basispolymere für positiv photostrukturierbare Zusammensetzungen gibt es keine Hinweise.

Aufgabe der Erfindung ist es, o-Aminophenol-carbonsäuren und o-Aminothiophenol-carbonsäuren bereitzustellen, die zur Herstellung von Polymeren geeignet sind, welche die stark gestiegenen Anforderungen der Mikroelektronik erfüllen. Die o-Amino(thio)phenol-carbonsäuren sollen insbesondere die Herstellung gut löslicher Polymer-Vorstufen ermöglichen, die nach der Cyclisierung auf einem Substrat Polybenzoxazole bzw. Polybenzothiazole mit geringer Feuchteaufnahme und hohem Planarisierungsgrad ergeben.

Dies wird erfindungsgemäß durch o-Aminophenol-carbonsäuren und o-Aminothiophenol-carbonsäuren folgender Struktur erreicht: 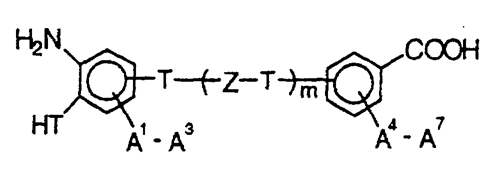 dabei gilt folgendes:
A¹ bis A⁷ sind - unabhängig voneinander - H, F, CH₃, CF₃, OCH₃, OCF₃, CH₂CH₃, CF₂CF₃, OCH₂CH₃ oder OCF₂CF₃,
wobei mindestens einer der Reste A¹ bis A³ F oder eine
F-haltige Gruppe sein muß;
T ist O oder S, und m ist 0 oder 1;
Z ist einer der folgenden Reste: 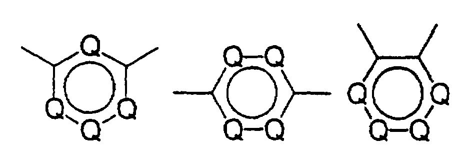 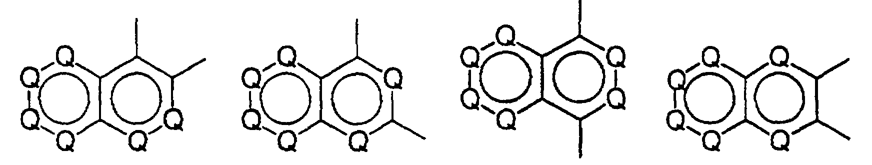 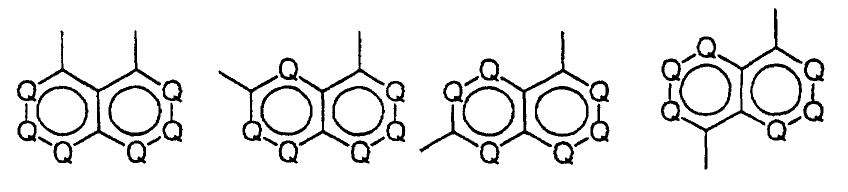 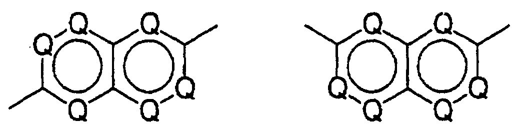 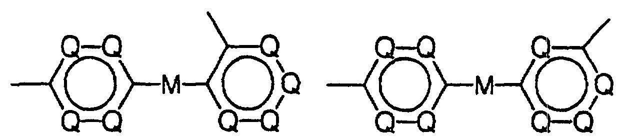 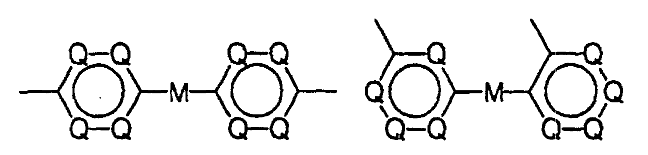 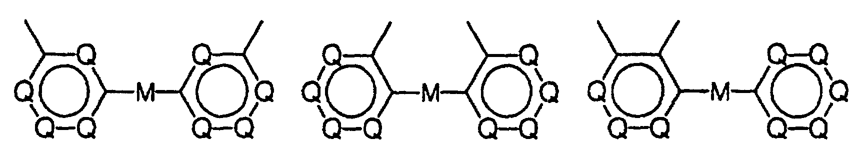 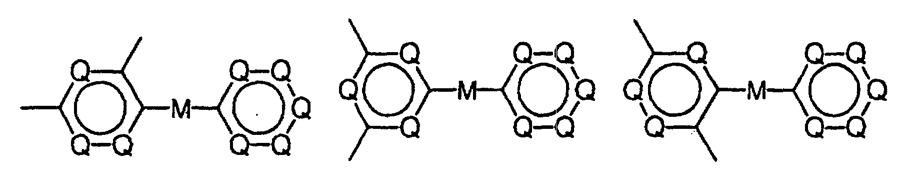 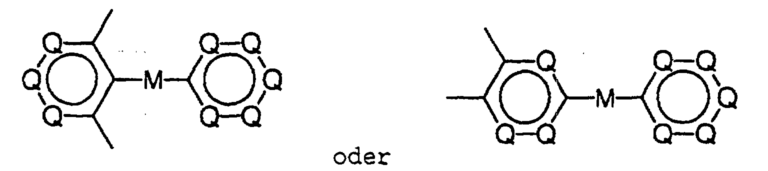
mit
- Q =: C-A oder N,
mit A = H, F, (CH₂)ₚCH₃, (CF₂)ₚCF₃, O(CH₂)ₚCH₃, O(CF₂)ₚCF₃, CO(CH₂)ₚCH₃, CO(CF₂)ₚCF₃ mit p = 0 bis 8 (Kette linear oder verzweigt), OC(CH₃)₃, OC(CF₃)₃, C₆H₅, C₆F₅, OC₆H₅, OC₆F₅, Cyclopentyl, Perfluorcyclopentyl, Cyclohexyl oder Perfluorcyclohexyl,
wobei in den isolierten aromatischen Ringen maximal 3 N-Atome pro Ring vorhanden sein dürfen und nur 2 N-Atome benachbart sein können und in den anellierten Ringsystemen maximal 2 N-Atome pro Ring vorhanden sein dürfen,
- M =: eine Einfachbindung, CH(CH₃), CH(CF₃), CF(CH₃), CF(CF₃), C(CH₃)₂, C(CF₃)₂, CH(C₆H₅), CH(C₆F₅), CF(C₆H₅), CF(C₆F₅), C(CH₃)(C₆H₅), C(CH₃)(C₆F₅), C(CF₃)(C₆H₅), C(CF₃)(C₆F₅), C(C₆H₅)₂, C(C₆F₅)₂, CO, SO₂,

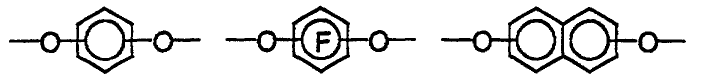

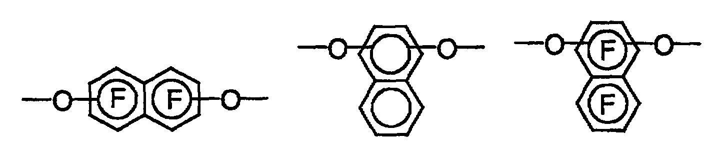

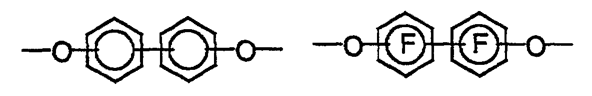

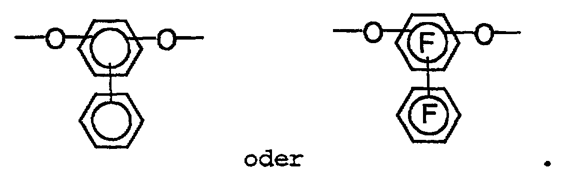

Die neuen Verbindungen weisen beispielsweise folgende Struktur auf:

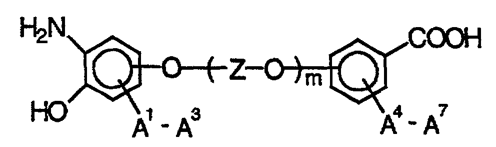

Bei derartigen Verbindungen sind offensichtlich die Etherbrücken für die gute Löslichkeit und die guten Planarisierungseigenschaften der damit hergestellten Polymer-Vorstufen verantwortlich. Im übrigen bedeutet die Charakterisierung "A¹-A³" und "A⁴-A⁷" in der Strukturformel, daß die Aminophenylgruppen Reste A¹, A² und A³ bzw. A⁴, A⁵, A⁶ und A⁷ aufweisen.

Die o-Amino(thio)phenol-carbonsäuren werden in der Weise hergestellt, daß
(a) eine halogenhaltige Nitroverbindung der Struktur 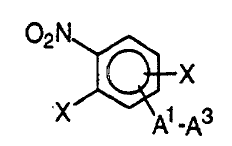 in einem Lösemittel bei einer Temperatur zwischen -10 und 80°C
   mit einer Hydroxy- bzw. Mercaptoverbindung der Struktur 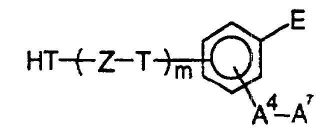 in Gegenwart mindestens der stöchiometrischen Menge einer Base oder mit einem Alkalisalz der Hydroxy- bzw. Mercaptoverbindung
   zur Reaktion gebracht wird,
   wobei X ein Halogenatom ist, E die Bedeutung CN oder COOR¹ mit R¹ = Alkyl (mit 1 bis 5 C-Atomen), Phenyl oder Benzyl hat und A¹ bis A⁷, T und Z wie angegeben definiert sind; und
(b) die dabei gebildete Nitroverbindung zur Aminoverbindung reduziert und hydrolysiert wird.

Bei diesem Syntheseverfahren, das sehr wirtschaftlich ist, wird somit zunächst eine Nitroverbindung mit wenigstens zwei Halogenatomen, von denen sich eines in o-Stellung zur Nitrogruppe befindet, mit einer Hydroxy- bzw. Mercaptoverbindung umgesetzt, die eine Ester- oder Nitrilgruppe aufweist. Das entstandene o-Nitro(thio)phenol wird dann zur entsprechenden Aminoverbindung reduziert, und die Ester- bzw. Nitrilgruppe wird zur Carboxylgruppe hydrolysiert.

Die Hydroxy- bzw. Mercaptoverbindung ist eine aromatische oder substituierte aromatische Verbindung. Für die Umsetzung mit der aromatischen Nitroverbindung sind prinzipiell alle Verbindungen geeignet, bei denen die Hydroxy- bzw. Mercaptogruppe eine ausreichende Nucleophilie besitzt.

Die Reaktion zwischen der Hydroxy- bzw. Mercaptoverbindung und der Nitroverbindung, bei der eine Ether- bzw. Thioetherbrücke gebildet wird, erfolgt in Gegenwart einer Base. Diese Base ist vorzugsweise ein Carbonat oder Hydrogencarbonat eines Alkali- oder Erdalkalimetalls, wie Natriumcarbonat und Kaliumcarbonat. Für die (Thio)etherbildung und für den Ersatz des Halogenatoms (in o-Stellung zur Nitrogruppe) durch eine Hydroxy- bzw. Mercaptogruppe ist jeweils mindestens die stöchiometrische Menge an der Base erforderlich. Vorteilhaft kann auch eine organische Base mit einem tertiären N-Atom, beispielsweise Triethylamin und Pyridin, eingesetzt werden. In diesem Fall ist die Zugabe von Wasser erforderlich. Anstelle der Hydroxy- bzw. Mercaptoverbindung kann auch ein entsprechendes Alkalisalz eingesetzt werden, beispielsweise das Kaliumsalz.

Als Reaktionstemperatur hat sich der Bereich von -10 bis 80°C als geeignet erwiesen. Temperaturen ≤ 80°C werden wegen der höheren Selektivität der Umsetzung bevorzugt. Vorteilhaft wird dabei in der Weise vorgegangen, daß zunächst für einige Zeit, beispielsweise ca. 16 h, eine Temperatur ≤ 25°C eingehalten wird, wobei die Umsetzung der Nitroverbindung mit der Hydroxy- bzw. Mercaptoverbindung erfolgt. Anschließend wird die Reaktion bei erhöhter Temperatur, d.h. ≥ 40°C, weitergeführt; hierbei erfolgt dann der Ersatz des Halogenatoms durch eine Hydroxy- bzw. Mercaptogruppe und die Hydrolyse der Ester- bzw. Nitrilgruppe. Bei dieser Vorgehensweise entstehen selektiv Produkte, bei denen sich die Hydroxy- bzw.

Mercaptogruppe in o-Stellung zur Nitrogruppe befindet. Bei der zweiten Stufe kann die Temperatur auch < 40°C sein, wenn die Temperaturdifferenz zur ersten Stufe ≥ 20°C ist.

Geeignete Lösemittel sind insbesondere Dimethylformamid, Diethylformamid, Dimethylacetamid, Dimethylsulfoxid, N-Methylpyrrolidon, γ-Butyrolacton, Acetonitril, Tetrahydrofuran und Pyridin. Prinzipiell können aber alle polaren aprotischen Lösemittel verwendet werden, in denen die Ausgangsverbindungen löslich sind.

Die Reduktion der Nitroverbindung kann beispielsweise durch Hydrierung mit Wasserstoff an Pd/C durchgeführt werden. Es sind aber prinzipiell alle Verfahren geeignet, die sich für die Reduktion der Nitrogruppe zur Aminogruppe eignen.

Die Reduktion der Nitroverbindung und die Hydrolyse der Ester- bzw. Nitrilgruppe kann in zwei getrennten Verfahrensschritten erfolgen, wobei die Hydrolyse beispielsweise mit Schwefelsäure durchgeführt wird; die Reihenfolge der Verfahrensschritte ist dabei beliebig. Beim Vorliegen einer Estergruppe erfolgt die Reduktion der Nitrogruppe und die Hydrolyse vorzugsweise jedoch gleichzeitig, und zwar durch Hydrierung mit Wasserstoff an Pd/C. Die Hydrierung erfolgt vorzugsweise bei Temperaturen von 25 bis 50°C. Geeignete Lösemittel sind Ester oder Ether, beispielsweise Essigsäureethylester und Tetrahydrofuran.

Alternativ können die o-Amino(thio)phenol-carbonsäuren auch in der Weise hergestellt werden, daß
(a) eine Halogenverbindung der Struktur 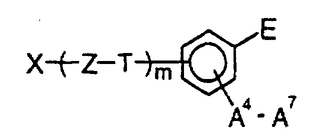 in einem Lösemittel bei einer Temperatur zwischen -10 und 80°C
   mit einem Nitrophenol bzw. Nitrothiophenol (kurz "Nitro-(thio)phenol") der Struktur 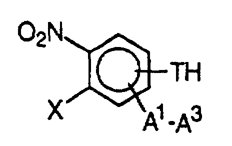 in Gegenwart mindestens der stöchiometrischen Menge einer Base oder mit einem Alkalisalz des Nitro(thio)phenols
   zur Reaktion gebracht wird,
   wobei X ein Halogenatom ist, E die Bedeutung CN oder COOR¹ mit R¹ = Alkyl (mit 1 bis 5 C-Atomen), Phenyl oder Benzyl hat und A¹ bis A⁷, T und Z wie angegeben definiert sind; und
(b) die dabei gebildete Nitroverbindung zur Aminoverbindung reduziert und hydrolysiert wird.

Bei diesem Syntheseverfahren, das ebenfalls sehr wirtschaftlich ist, wird somit ein halogenhaltiger Ester oder ein entsprechendes Nitril mit einem Nitro(thio)phenol umgesetzt, das in o-Stellung zur Nitrogruppe ein Halogenatom aufweist. Die dabei gebildete Nitroverbindung wird dann zur entsprechenden Aminoverbindung reduziert und die Ester- bzw. Nitrilgruppe wird zur Carboxylgruppe hydrolysiert.

Die aus den o-Amino(thio)phenol-carbonsäuren nach der Erfindung hergestellten Polymer-Vorstufen, die - im Vergleich zum Stand der Technik - verbesserte Eigenschaften aufweisen, sind in vielen organischen Lösemitteln, wie Aceton, Cyclohexanon, N-Methylpyrrolidon, Diethylenglykolmono- bzw. -diethylether, Ethyllactat und γ-Butyrolacton, sowie in metallionenfreien wäßrig-alkalischen Entwicklern gut löslich. Sie sind deshalb als Basispolymer für positiv photostrukturierbare und wäßrigalkalisch entwickelbare Dielektrika gut geeignet. Die Vorstufen können mittels Schleudertechnik problemlos auf Substrate, wie Siliziumwafer, aufgebracht werden, sie bilden gleichmäßige Filme und lassen sich auf dem Substrat gut cyclisieren. Ein besonderer Vorteil der aus diesen o-Amino-(thio)phenol-carbonsäuren hergestellten Vorstufen ist das hohe Planarisierungsvermögen und die geringe Feuchteaufnahme.

Anhand von Ausführungsbeispielen soll die Erfindung noch näher erläutert werden.

### Beispiel 1

Herstellung von 4-(4-Nitro-3-hydroxy-2,5,6-trifluor-phenoxy)-benzoesäurebenzylester 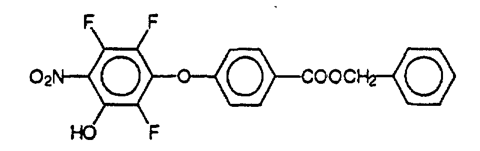 42,6 g Pentafluor-nitrobenzol (0,2 mol) werden in 300 ml N-Methylpyrrolidon gelöst. Nach der Zugabe von 30 g Kaliumcarbonat (0,22 mol) wird auf -5°C ab gekühlt und dann wird innerhalb von 30 min unter Rühren eine Lösung von 45,6 g 4-Hydroxy-benzoesäurebenzylester (0,2 mol) in 300 ml N-Methylpyrrolidon zugetropft. Nach 1 h wird die Reaktionstemperatur für 24 h auf 23°C erhöht, dann gibt man 800 ml Wasser und 300 ml Essigsäureethylester zu. Die organische Phase wird abgetrennt, dreimal mit Wasser gewaschen und über Natriumsulfat getrocknet. Die Lösung wird dann in einem Rotationsverdampfer eingeengt, bis gelbe Kristalle ausfallen.

Das Reaktionsprodukt wird mit Petroleumbenzin (Siedebereich 60° bis 80°C) gewaschen, über einen Büchnertrichter abfiltriert und anschließend in einem Vakuumschrank 48 h unter Stickstoff bei 40°C/10 mbar getrocknet (Ausbeute: 89 %).

### Charakterisierung:

- Massenspektrum: Molekülpeak bei 419
- Elementaranalyse:

| | | | |
|---|---|---|---|
| Theoretischer Wert (in %) | C: 57,3 | H: 2,9 | N: 3,3 |
| Ermittelter Wert (in %) | C: 57,5 | H: 3,0 | N: 3,3 |

- Schmp.: 78°C.

### Beispiel 2

Herstellung von 4-(4-Amino-3-hydroxy-2,5,6-trifluor-phenoxy)-benzoesäure 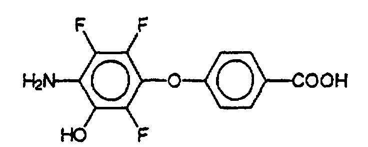 50 g des entsprechend Beispiel 1 hergestellten 4-(4-Nitro-3-hydroxy-2,5,6-trifluor-phenoxy)-benzoesaurebenzylester (0,12 mol) werden in 600 ml eines Gemisches von Tetrahydrofuran und Essigsäureethylester (Volumenverhältnis 1:1) gelöst, und zu der Lösung werden 5 g Pd/C (Palladium/Kohlenstoff) gegeben. Dann wird bei Raumtemperatur in einem Autoklaven unter starkem Rühren mit Wasserstoff bei einem Druck von 1 bar hydriert; nach 3 Tagen wird die Reaktion beendet. Die gelbe Lösung wird in einem Rotationsverdampfer auf die Hälfte eingeengt und über Nacht bei Raumtemperatur stehengelassen; dabei fällt das Reaktionsprodukt kristallin aus. Anschließend wird das Reaktionsprodukt in einem Vakuumschrank 48 h unter Stickstoff bei 40°C/10 mbar getrocknet (Ausbeute: 93 %).

Charakterisierung:
- Massenspektrum: Molekülpeak bei 299
- Elementaranalyse:

| | | | |
|---|---|---|---|
| Theoretischer Wert (in %) | C: 52,2 | H: 2,7 | N: 4,7 |
| Ermittelter Wert (in %) | C: 52,1 | H: 2,7 | N: 4,6 |

### Beispiel 3

Herstellung von 4-(4-Benzyloxycarbonyl-phenoxy)-nonafluorbiphenyl 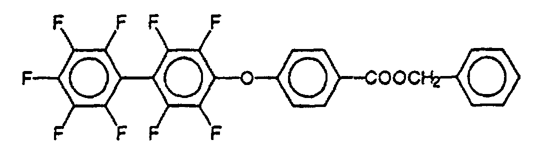 37,6 g Decafluorbiphenyl (0,112 mol) werden in 700 ml Dimethylformamid gelöst und mittels eines Kryostaten auf -10°C abgekühlt, dann wird innerhalb von 2 h eine Lösung von 15 g Kalium-4-benzyloxycarbonylphenolat (0,056 mol) in 300 ml Dimethylformamid zugetropft. Nach 48 h bei -10°C ist das Kaliumsalz umgesetzt. Dann wird das Dimethylformamid in einem Rotationsverdampfer entfernt, der Rückstand in wenig Tetrahydrofuran aufgenommen und über eine Kieselgelsäule filtriert. Die erhaltene klare Lösung wird im Rotationsverdampfer eingeengt, bis ein weißer Feststoff ausfällt. Der Feststoff wird dann in n-Hexan gerührt, über ein Faltenfilter abfiltriert und anschließend in einem Vakuumschrank 48 h unter Stickstoff bei 40°C/10 mbar getrocknet (Ausbeute: 91 %).

Charakterisierung:
- Massenspektrum: Molekülpeak bei 542
- Elementaranalyse:

| | | |
|---|---|---|
| Theoretischer Wert (in %) | C: 57,6 | H: 2,0 |
| Ermittelter Wert (in %) | C: 57,5 | H: 1,9 |

- Schmp: 120°C.

### Beispiel 4

Herstellung von 4-Nitro-tetrafluorphenol 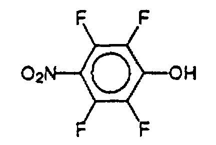 21,3 g Pentafluor-nitrobenzol (0,1 mol) werden in 400 ml Dimethylsulfoxid gelöst; unter starkem Rühren wird dazu eine Lösung von 11,2 g Kaliumhydroxid (0,2 mol) in 100 ml Wasser getropft. Nach 24 h bei Raumtemperatur wird das Rohprodukt mit 200 ml Essigsäureethylester und 400 ml Wasser ausgeschüttet. Die organische Phase wird dreimal mit Wasser gewaschen, über Natriumsulfat getrocknet und in einem Rotationsverdampfer auf die Hälfte eingeengt. Aus der anfangs zähen Lösung kristallisiert das Reaktionsprodukt aus. Das Reaktionsprodukt wird dann in einem Gemisch von n-Hexan und Methylenchlorid (Volumenverhältnis 1:1) umkristallisiert und anschließend in einem Vakuumschrank 48 h unter Stickstoff bei 40°C/10 mbar getrocknet (Ausbeute: 95 %).

Charakterisierung:
- Massenspektrum: Molekülpeak bei 211
- Elementaranalyse:

| | | | |
|---|---|---|---|
| Theoretischer Wert (in %) | C: 34,1 | H: 0,5 | N: 6,6 |
| Ermittelter Wert (in %) | C: 34,0 | H: 0,4 | N: 6,7 |

- Schmp: 171°C.

### Beispiel 5

Herstellung von 4-(4-Nitro-3-hydroxy-2,5,6-trifluor-phenoxy)-4'-(4-benzyloxycarbonyl-phenoxy)-octafluorbiphenyl 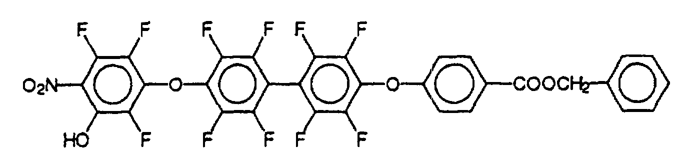 50 g des entsprechend Beispiel 3 hergestellten 4-(4-Benzyl-oxycarbonyl-phenoxy)-nonafluorbiphenyl (0,092 mol) und 19,4 g des entsprechend Beispiel 4 hergestellten 4-Nitro-tetrafluorphenol (0,092 mol) werden in 400 ml Dimethylsulfoxid gelöst. Zur Lösung werden portionsweise 25 g Kaliumcarbonat (0,184 mol) gegeben, dann wird 24 h bei Raumtemperatur gerührt. Anschließend wird 24 h auf 60°C erhitzt, und dann werden 10 g Kaliumhydrogencarbonat (0,1 mol) zugegeben. Danach läßt man die Reaktionslösung auf Raumtemperatur abkühlen und filtriert über ein Faltenfilter ab. Das Rohprodukt wird mit 300 ml Essigsäureethylester und 700 ml Wasser ausgeschüttelt, die organische Phase dreimal mit Wasser gewaschen und in einem Rotationsverdampfer eingeengt, bis das Reaktionsprodukt ausfällt. Das Reaktionsprodukt wird dann in einem Gemisch von Essigsäureethylester und n-Hexan (Volumenverhältnis 1:1) umkristallisiert und anschließend in einem Vakuumschrank 48 h unter Stickstoff bei 40°C/10 mbar getrocknet (Ausbeute: 92 %).

Charakterisierung:
- Massenspektrum: Molekülpeak bei 731
- Elementaranalyse:

| | | | |
|---|---|---|---|
| Theoretischer Wert (in %) | C: 52,5 | H: 1,7 | N: 1,9 |
| Ermittelter Wert (in %) | C: 52,7 | H: 1,8 | N: 1,8 |

### Beispiel 6

Herstellung von 4-(4-Amino-3-hydroxy-2,5,6-trifluor-phenoxy)-4'-(4-carboxy-phenoxy)-octafluorbiphenyl 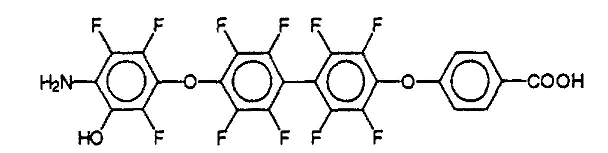 50 g des entsprechend Beispiel 5 hergestellten 4-(4-Nitro-3-hydroxy-2,5,6-trifluor-phenoxy)-4'-(4-benzyloxycarbonylphenoxy)-octafluorbiphenyl (0,069 mol) werden in 600 ml eines Gemisches von Tetrahydrofuran und Essigsäureethylester (Volumenverhältnis 1:1) gelöst, und zu der Lösung werden 5 g Pd/C (Palladium/Kohlenstoff) gegeben. Dann wird bei Raumtemperatur in einem Autoklaven unter starkem Rühren mit Wasserstoff bei einem Druck von 1 bar hydriert; nach 3 Tagen wird die Reaktion beendet. Die orangefarbene Lösung wird in einem Rotationsverdampfer auf die Hälfte eingeengt und über Nacht bei Raumtemperatur stehengelassen; dabei fällt das Reaktionsprodukt kristallin aus. Anschließend wird das Reaktionsprodukt in einem Vakuumschrank 48 h unter Stickstoff bei 40°C/10 mbar getrocknet (Ausbeute: 93 %).

Charakterisierung:
- Massenspektrum: Molekülpeak bei 611
- Elementaranalyse:

| | | | |
|---|---|---|---|
| Theoretischer Wert (in %) | C: 49,1 | H: 1,3 | N: 2,3 |
| Ermittelter Wert (in %) | C: 48,9 | H: 1,4 | N: 2,3 |

### Beispiel 7

Herstellung von 2-(4-Benzyloxycarbonyl-phenoxy)-3,4,5,6-tetrafluorpyridin 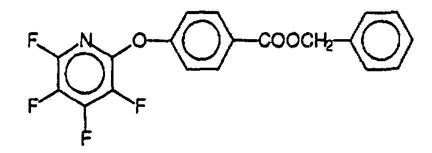 33,8 g Pentafluorpyridin (0,2 mol) werden in 500 ml Dimethylformamid gelöst und mittels eines Kryostaten auf 0°C abgekühlt, dann wird innerhalb von 2 h eine Lösung von 53,6 g Kalium-4-benzyloxycarbonylphenolat (0,2 mol) in 400 ml Dimethylformamid zugetropft. Nach 24 h bei 0°C ist das Kaliumsalz umgesetzt. Dann wird das Dimethylformamid in einem Rotationsverdampfer entfernt, der Rückstand in wenig Tetrahydrofuran aufgenommen und über eine Kieselgelsäule filtriert. Die erhaltene klare Lösung wird im Rotationsverdampfer eingeengt, bis das Reaktionsprodukt ausfällt. Das Reaktionsprodukt wird dann in n-Hexan gerührt, über ein Faltenfilter abfiltriert und anschließend in einem Vakuumschrank 48 h unter Stickstoff bei 40°C/10 mbar getrocknet (Ausbeute: 91 %).

Charakterisierung:
- Massenspektrum: Molekülpeak bei 377
- Elementaranalyse:

| | | | |
|---|---|---|---|
| Theoretischer Wert (in %) | C: 60,5 | H: 2,9 | N: 3,7 |
| Ermittelter Wert (in %) | C: 60,6 | H: 2,9 | N: 3,6 |

### Beispiel 8

Herstellung von 4-(4-Nitro-3-hydroxy-2,5,6-trifluor-phenoxy)-2-(4-benzyloxycarbonyl-phenoxy)-3,5,6-trifluorpyridin 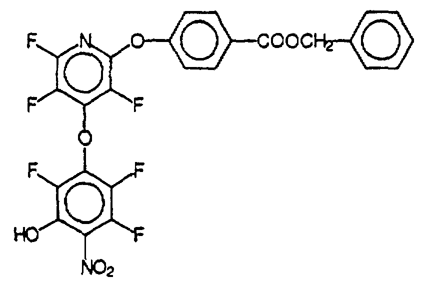 40 g des entsprechend Beispiel 7 hergestellten 2-(4-Benzyloxycarbonyl-phenoxy)-3,4,5,6-tetrafluorpyridin (0,106 mol) und 22,4 g des entsprechend Beispiel 4 hergestellten 4-Nitrotetrafluorphenol (0,106 mol) werden in 400 ml Dimethylsulfoxid gelöst. Zur Lösung werden portionsweise 30 g Kaliumcarbonat (0,22 mol) gegeben, dann wird 24 h bei Raumtemperatur gerührt. Anschließend wird 24 h auf 60°C erhitzt, und dann werden 15 g Kaliumhydrogencarbonat (0,15 mol) zugegeben. Danach läßt man die Reaktionslösung auf Raumtemperatur abkühlen und filtriert über ein Faltenfilter ab. Das Rohprodukt wird mit 300 ml Essigsäureethylester und 700 ml Wasser ausgeschüttelt, die organische Phase dreimal mit Wasser gewaschen und in einem Rotationsverdampfer eingeengt, bis das Reaktionsprodukt ausfällt. Das Reaktionsprodukt wird dann in einem Gemisch von Essigsäureethylester und n-Hexan (Volumenverhältnis 1:1) umkristallisiert und anschließend in einem Vakuumschrank 48 h unter Stickstoff bei 40°C/10 mbar getrocknet (Ausbeute: 92 %).

Charakterisierung:
- Massenspektrum: Molekülpeak bei 566
- Elementaranalyse:

| | | | |
|---|---|---|---|
| Theoretischer Wert (in %) | C: 53,0 | H: 2,1 | N: 4,9 |
| Ermittelter Wert (in %) | C: 52,8 | H: 2,1 | N: 5,0 |

### Beispiel 9

Herstellung von 4-(4-Amino-3-hydroxy-2,5,6-trifluor-phenoxy)-2-(4-carboxy-phenoxy)-3,5,6-trifluorpyridin 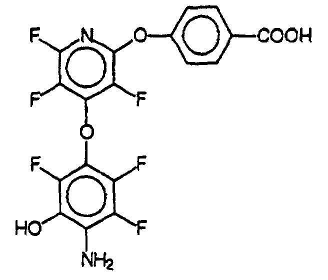 35 g des entsprechend Beispiel 8 hergestellten 4-(4-Nitro-3-hydroxy-2,5,6-trifluor-phenoxy)-2-(4-benzyloxycarbonylphenoxy)-3,5,6-trifluorpyridin (0,062 mol) werden in 500 ml eines Gemisches von Tetrahydrofuran und Essigsäureethylester (Volumenverhältnis 1:1) gelöst, und zu der Lösung werden 3,5 g Pd/C (Palladium/Kohlenstoff) gegeben. Dann wird bei Raumtemperatur in einem Autoklaven unter starkem Rühren mit Wasserstoff bei einem Druck von 1 bar hydriert; nach 2 Tagen wird die Reaktion beendet. Die gelbe Lösung wird in einem Rotationsverdampfer auf die Hälfte eingeengt und über Nacht bei Raumtemperatur stehengelassen; dabei fällt das Reaktionsprodukt kristallin aus. Anschließend wird das Reaktionsprodukt in einem Vakuumschrank 48 h unter Stickstoff bei 40°C/10 mbar getrocknet (Ausbeute: 91 %).

Charakterisierung:
- Massenspektrum: Molekülpeak bei 446
- Elementaranalyse:

| | | | |
|---|---|---|---|
| Theoretischer Wert (in %) | C: 48,4 | H: 1,8 | N: 6,3 |
| Ermittelter Wert (in %) | C: 48,5 | H: 1,7 | N: 6,3 |

## Patentansprüche

1. o-Aminophenol-carbonsäuren und o-Aminothiophenol-carbonsäuren der Struktur 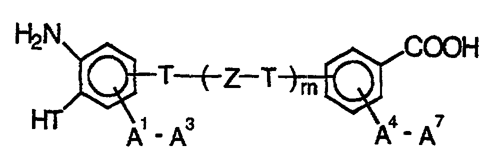 wobei folgendes gilt:
A¹ bis A⁷ sind - unabhängig voneinander - H, F, CH₃, CF₃, OCH₃, OCF₃, CH₂CH₃, CF₂CF₃, OCH₂CH₃ oder OCF₂CF₃,
wobei mindestens einer der Reste A¹ bis A³ F oder eine
F-haltige Gruppe sein muß;
T ist O oder S, und m ist 0 oder 1;
Z ist einer der folgenden Reste: 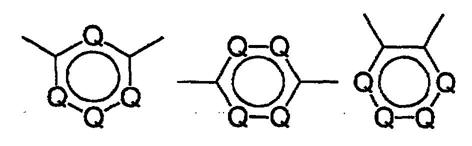 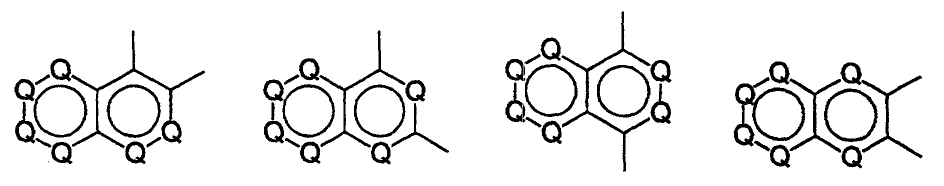 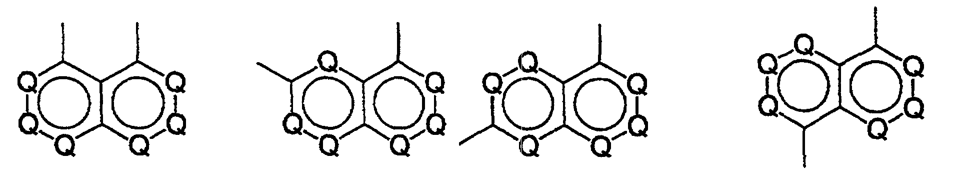 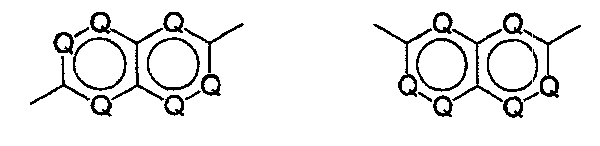 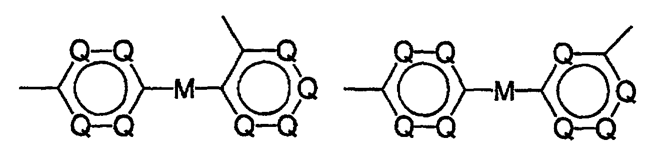 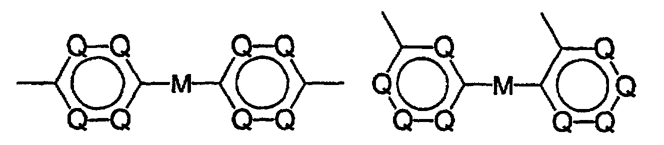 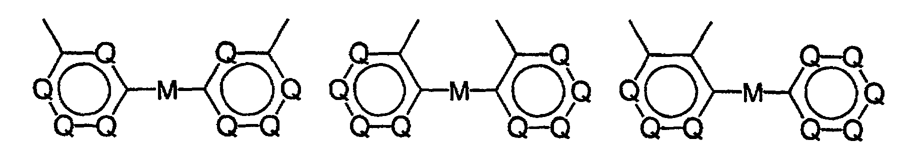 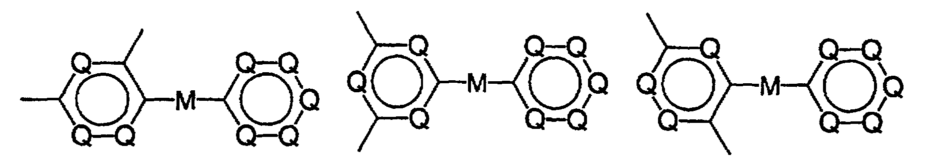 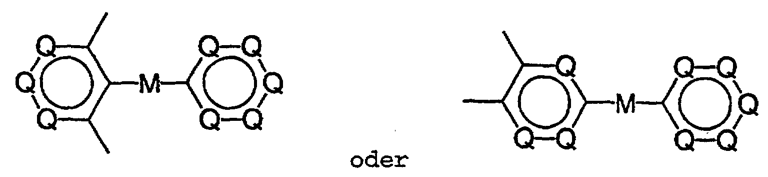
mit
Q = C-A oder N,
mit A = H, F, (CH₂)ₚCH₃, (CF₂)ₚCF₃, O(CH₂)ₚCH₃, O(CF₂)ₚCF₃, CO(CH₂)ₚCH₃, CO(CF₂)ₚCF₃ mit p = 0 bis 8 (Kette linear oder verzweigt), OC(CH₃)₃, OC(CF₃)₃, C₆H₅, C₆F₅, OC₆H₅, OC₆F₅, Cyclopentyl, Perfluorcyclopentyl, Cyclohexyl oder Perfluorcyclohexyl, wobei in den isolierten aromatischen Ringen maximal 3 N-Atome pro Ring vorhanden sein dürfen und nur 2 N-Atome benachbart sein können und in den anellierten Ringsystemen maximal 2 N-Atome pro Ring vorhanden sein dürfen,
M = eine Einfachbindung, CH(CH₃), CH(CF₃), CF(CH₃), CF(CF₃), C(CH₃)₂, C(CF₃)₂, CH(C₆H₅), CH(C₆F₅), CF(C₆H₅), CF(C₆F₅), C(CH₃) (C₆H₅), C(CH₃)(C₆F₅), C(CF₃)(C₆H₅), C(CF₃)(C₆F₅), C(C₆H₅)₂, C(C₆F₅)₂, CO, SO₂,

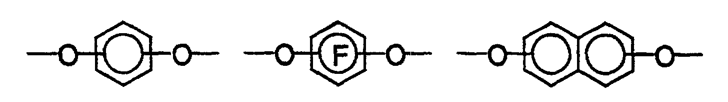

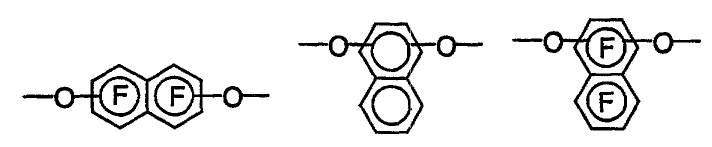

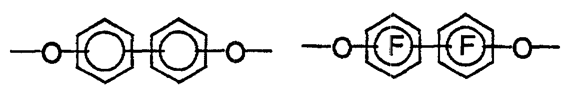

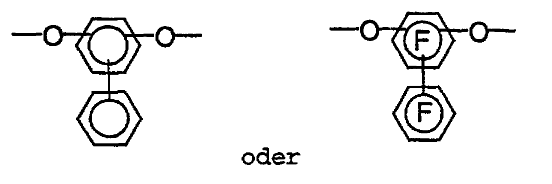

2. Verfahren zur Herstellung von o-Aminophenol-carbonsäuren und o-Aminothiophenol-carbonsäuren nach Anspruch 1, **dadurch gekennzeichnet, daß**
(a) eine halogenhaltige Nitroverbindung der Struktur 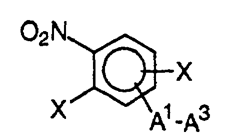 in einem Lösemittel bei einer Temperatur zwischen -10 und 80°C
mit einer Hydroxy- bzw. Mercaptoverbindung der Struktur 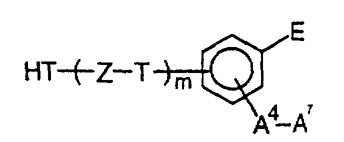 in Gegenwart mindestens der stöchiometrischen Menge einer Base oder mit einem Alkalisalz der Hydroxy- bzw. Mercaptoverbindung
zur Reaktion gebracht wird,
wobei X ein Halogenatom ist, E die Bedeutung CN oder COOR¹ mit R¹ = Alkyl (mit 1 bis 5 C-Atomen), Phenyl oder Benzyl hat und A¹ bis A⁷, T und Z wie angegeben definiert sind; und
(b) die dabei gebildete Nitroverbindung zur Aminoverbindung reduziert und hydrolysiert wird.

3. Verfahren zur Herstellung von o-Aminophenol-carbonsäuren und o-Aminothiophenol-carbonsäuren nach Anspruch 1, **dadurch gekennzeichnet, daß**
(a) eine Halogenverbindung der Struktur 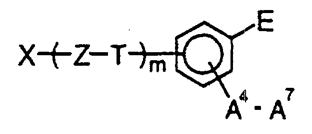 in einem Lösemittel bei einer Temperatur zwischen -10 und 80°C
mit einem Nitrophenol bzw. Nitrothiophenol der Struktur 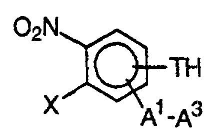 in Gegenwart mindestens der stöchiometrischen Menge einer Base oder mit einem Alkalisalz des Nitro(thio)phenols zur Reaktion gebracht wird,
wobei X ein Halogenatom ist, E die Bedeutung CN oder
COOR¹ mit R¹ = Alkyl (mit 1 bis 5 C-Atomen), Phenyl oder Benzyl hat und A¹ bis A⁷, T und Z wie angegeben definiert sind; und
(b) die dabei gebildete Nitroverbindung zur Aminoverbindung reduziert und hydrolysiert wird.

4. Verfahren nach Anspruch 2 oder 3, **dadurch gekennzeichnet, daß** als Base ein Carbonat oder Hydrogencarbonat eines Alkali- oder Erdalkalimetalls eingesetzt wird.

5. Verfahren nach Anspruch 2 oder 3, **dadurch gekennzeichnet, daß** eine organische Base mit einem tertiären N-Atom zusammen mit Wasser eingesetzt wird.

6. Verfahren nach einem der Ansprüche 2 bis 5, **dadurch gekennzeichnet, daß** die Reduktion sowie - bei E = COOR¹ - die Hydrolyse durch Wasserstoff erfolgt und mit Pd/C katalysiert wird.

## Claims

1. o-Aminophenolcarboxylic acids or o-aminothiophenolcarboxylic acids of the structure 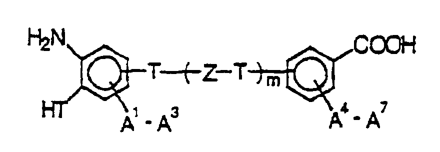 where:
A¹ to A⁷ are - independently of one another-H, F, CH₃, CF₃, OCH₃, OCF₃, CH₂CH₃, CF₂CF₃, OCH₂CH₃ or OCF₂CF₃, where at least one of the radicals A¹ to A³ must be F or an F-containing group;
T is O or S, and m is 0 or 1;
Z is one of the following radicals: 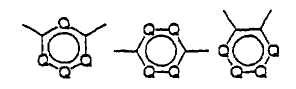 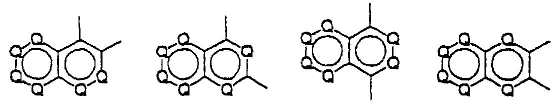 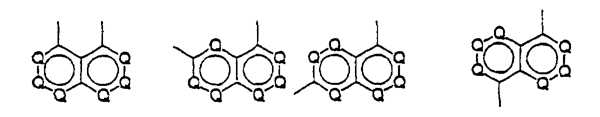 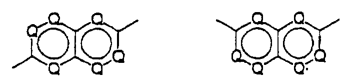 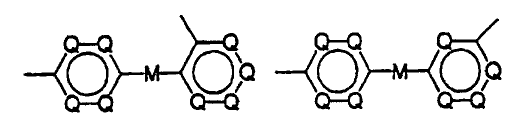 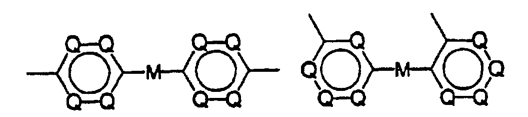 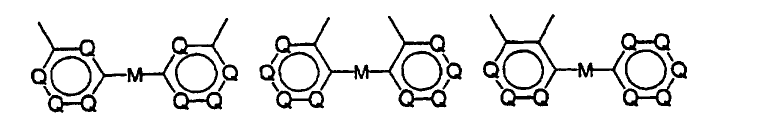 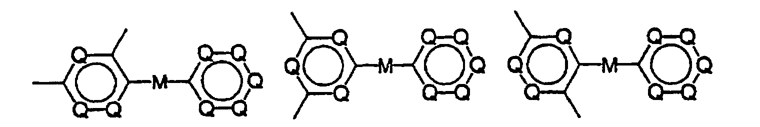 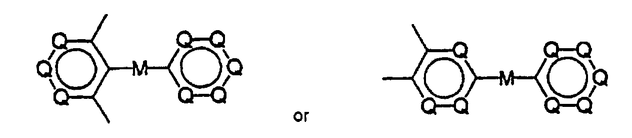 where
Q = C-A or N,
where A = H, F, (CH₂)ₚCH₃, (CF₂)ₚCF₃, O(CH₂)ₚCH₃, O(CF₂)ₚCF₃, CO(CH₂)ₚCH₃, CO(CF₂)ₚCF₃, where p = 0 to 8 (linear or branched chain), OC(CH₃)₃, OC(CF₃)₃, C₆H₅, C₆F₅, OC₆H₅, OC₆F₅, cyclopentyl, perfluorocyclopentyl, cyclohexyl or perfluorocyclohexyl,
where, in the isolated aromatic rings, a maximum of 3 N atoms may be present per ring and only 2 N atoms may be adjacent, and, in the fused ring systems, a maximum of 2 N atoms may be present per ring,
M = a single bond, CH(CH₃), CH(CF₃), CF(CH₃), CF(CF₃), C(CH₃)₂, C(CF₃)₂, CH(C₆H₅), CH(C₆F₅), CF(C₆H₅), CF(C₆F₅), C(CH₃)(C₆H₅), C(CH₃)(C₆F₅), C(CF₃)(C₆H₅), C(CF₃)(C₆F₅), C(C₆H₅)₂, C(C₆F₅)₂, CO, SO₂,

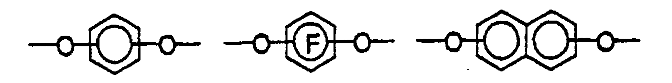

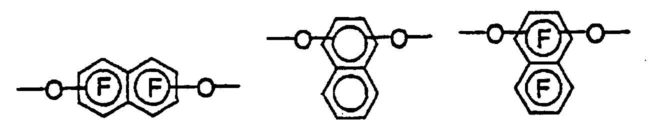

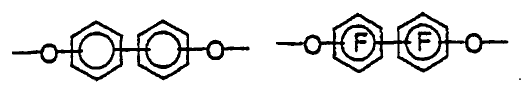

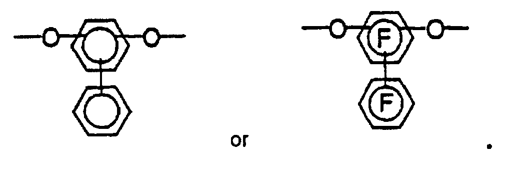

2. Process for the preparation of o-aminophenolcarboxylic acids or o-aminothiophenolcarboxylic acids according to Claim 1, **characterised in that**
(a) a halogen-containing nitro compound of the structure 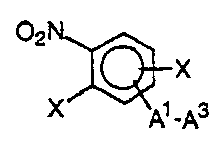 is reacted with a hydroxy or mercapto compound of the structure 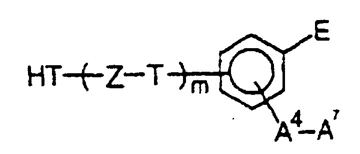 in the presence of at least the stoichiometric amount of a base, or with an alkali metal salt of the hydroxy or mercapto compound, in a solvent at a temperature between -10 and 80°C,
where X is a halogen atom, E is CN or COOR¹, where R¹ = alkyl (having 1 to 5 carbon atoms), phenyl or benzyl, and A¹ to A⁷, T and Z are as defined above, and
(b) the resultant nitro compound is reduced to the amino compound and the latter is hydrolysed.

3. Process for the preparation of o-aminophenolcarboxylic acids or o-aminothiophenolcarboxylic acids according to Claim 1, **characterised in that**
(a) a halogen compound of the structure 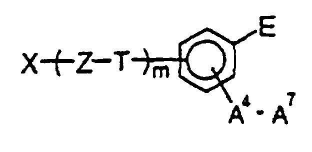 is reacted with a nitrophenol or nitrothiophenol of the structure 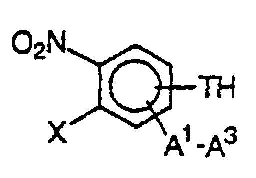 in the presence of at least the stoichiometric amount of a base, or with an alkali metal salt of the nitro(thio)phenol, in a solvent at a temperature between -10 and 80°C,
where X is a halogen atom, E is CN or COOR¹, where R¹=alkyl (having 1 to 5 carbon atoms), phenyl or benzyl, and A¹ to A⁷, T and Z are as defined above; and
(b) the resultant nitro compound is reduced to the amino compound and the latter is hydrolysed.

4. Process according to Claim 2 or 3, the base used is a carbonate or hydrogencarbonate of an alkali metal or alkaline earth metal.

5. Process according to Claim 2 or 3, **characterised in that** an organic base containing a tertiary N atom is used together with water.

6. Process according to any one of Claims 2 to 5, **characterised in that** the reduction and - if E = COOR¹ - the hydrolysis are carried out by means of hydrogen and are catalysed by Pd/C.

## Revendications

1. Acides o-aminophénol carboxyliques et acides oaminothiophénol carboxyliques de formule : 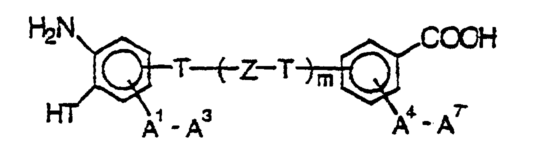 dans laquelle :
A¹ à A⁷ sont indépendamment les uns des autres H, F, CH₃, CF₃, OCH₃, OCF₃, CH₂CH₃, CF₂CF₃, OCH₂CH₃ ou OCF₂CF₃,
l'un au moins des radicaux A¹ à A³ devant être F ou un groupe contenant F ;
T est O ou S et m est 0 ou 1 ;
Z est l'un des radicaux suivants : 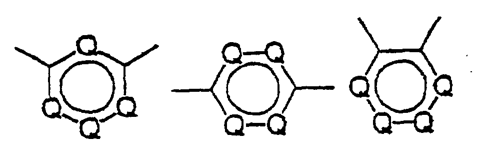 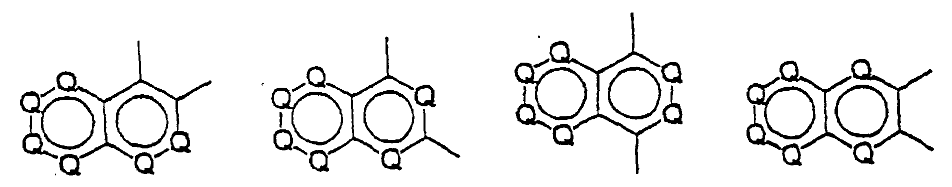 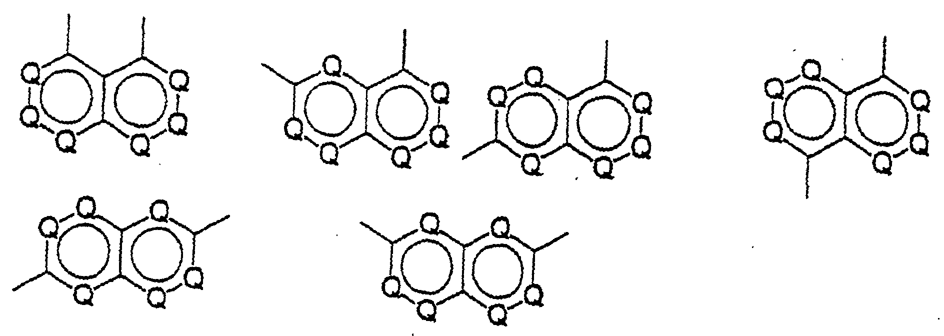 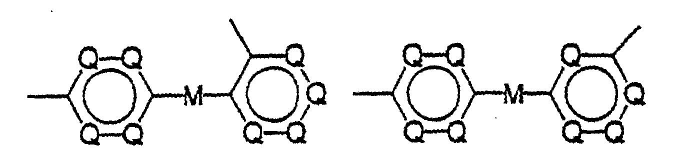 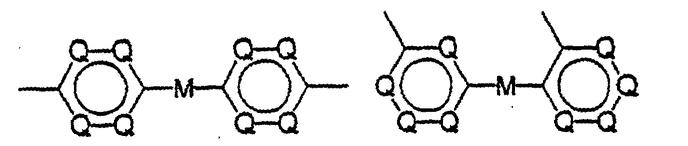 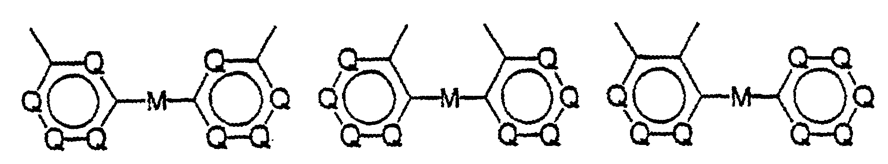 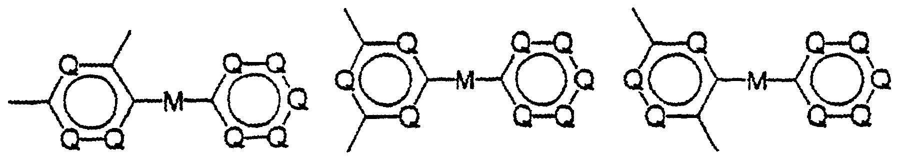 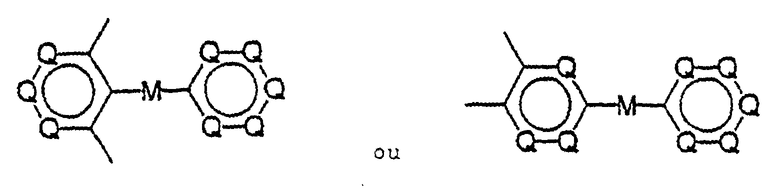
Q = C-A ou N,
avec A = H, F, (CH₂)ₚCH₃, (CF₂)ₚCF₃, O(CH₂)ₚCH₃, O(CF₂)ₚCF₃, CO(CH₂)ₚCH₃, CO(CF₂)ₚCF₃ avec p = 0 à 8 (chaîne linéaire ou ramifiée), OC(CH₃)₃, OC(CF₃)₃, C₆H₅, C₆F₅, OC₆H₅, OC₆F₅, cyclopentyle, perfluorocyclopentyle, cyclohexyle ou perfluorocyclohexyle, et il peut y avoir dans les cycles aromatiques isolés au maximum 3 atomes d'azote par cycle et seulement 2 atomes d'azote peuvent être voisins et il peut y avoir dans les systèmes cycliques condensés au maximum 2 atomes d'azote par cycle,
M = simple liaison, CH(CH₃), CH(CF₃), CF(CH₃), CF(CF₃), C(CH₃)₂, C(CF₃)₂, CH(C₆H₅), CH(C₆F₅), CF(C₆H₅), CF(C₆F₅), C(CH₃)(C₆H₅), C(CH₃)(C₆F₅), C(CF₃)(C₆H₅), C(CF₃)(C₆F₅), C(C₆H₅)₂, C(C₆F₅)₂, CO, SO₂,

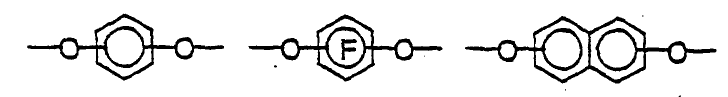

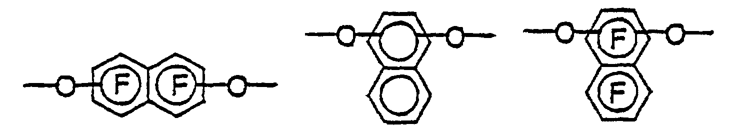

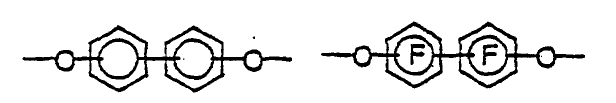

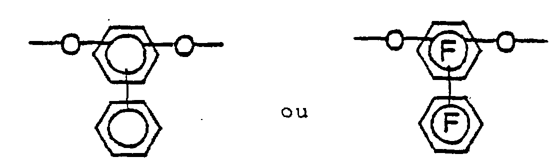

2. Procédé de préparation d'acides o-aminophénol carboxyliques et d'acides o-aminothiophénol carboxyliques suivant la revendication 1, **caractérisé en ce qu'**il consiste à mettre
(a) un composé nitro halogéné de formule 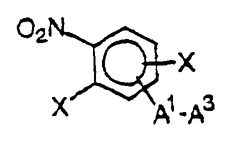 à réagir dans un solvant à une température comprise entre -10 et 80°C sur un composé hydroxy ou sur un composé mercapto de formule 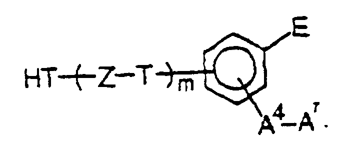 en présence d'au moins la quantité stoechiométrique d'une base ou sur un sel de métal acalin du composé hydroxy ou du composé mercapto,
X étant un atome d'halogène, E ayant la signification CN ou COOR¹ avec R¹ = alcoyle (ayant de 1 à 5 atomes de carbone), phényle ou benzyle et A¹ à A⁷, T et Z étant définis comme indiqué ; et
(b) à réduire et à hydrolyser le composé nitro ainsi formé en un composé amino.

3. Procédé de préparation d'acides o-aminophénol carboxyliques et d'acides o-aminothiophénol carboxyliques suivant la revendication 1,
**caractérisé en ce qu'**il consiste
(a) à mettre un composé halogéné de formule 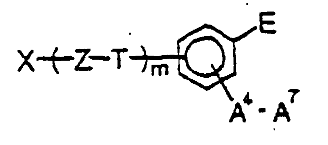 à réagir dans un solvant à une température comprise entre -10 et 80°C sur un nitrophénol ou sur un nitrothiophénol de formule 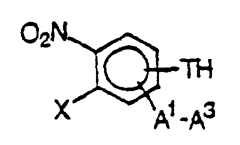 en présence d'au moins la quantité stoechiométrique d'une base ou sur un sel de métal acalin du nitro(thio)phénol
X étant un atome d'halogène, E ayant la signification CN ou COOR¹ avec R¹ = alcoyle (ayant de 1 à 5 atomes de carbone), phényle ou benzyle et A¹ à A⁷, T et Z étant définis comme indiqué ; et
(b) à réduire et à hydrolyser le composé nitro ainsi formé en le composé amino.

4. Procédé suivant la revendication 2 ou 3, **caractérisé en ce qu'**il consiste à utiliser comme base un carbonate ou un hydrogénocarbonate d'un métal alcalin ou alcalinoterreux.

5. Procédé suivant la revendication 2 ou 3, **caractérisé en ce qu'**il consiste à utiliser une base organique ayant un atome d'azote tertiaire en même temps que de l'eau.

6. Procédé suivant l'une des revendications 2 à 5, **caractérisé en ce qu'**il consiste à effectuer la réduction, ainsi que dans le cas où E = COOR¹ l'hydrolyse, par de l'hydrogène et à catalyser par Pd/C.
